# EUROPÄISCHE PATENTSCHRIFT

(11) **EP 1 159 236 B1**
(45) Veröffentlichungstag und Bekanntmachung des Hinweises auf die Patenterteilung: **29.10.2003**
(21) Anmeldenummer: 00907630.8
(22) Anmeldetag: 25.02.2000
(51) Int. Cl.: C07C 2/10

(54) **VERFAHREN ZUR OLIGOMERISIERUNG VON C6-OLEFINEN**
METHOD FOR OLIGOMERIZING C6 OLEFINS
PROCEDE D'OLIGOMERISATION D'OLEFINES C6

(30) Priorität: 08.03.1999 DE 19910103
(43) Veröffentlichungstag der Anmeldung: 05.12.2001
(73) Patentinhaber: BASF AKTIENGESELLSCHAFT, 67056 Ludwigshafen (DE)
(72) Erfinder: MAAS, Heiko, D-67105 Schifferstadt (DE); SCHWAB, Peter, D-67098 Bad Dürkheim (DE); SCHULZ, Ralf, D-67346 Speyer (DE); WALTER, Marc, D-67227 Frankenthal (DE); BROX, Wolfgang, D-69118 Heidelberg (DE)
(74) Vertreter: Isenbruck, Günter, Dr.
(86) Internationale Anmeldenummer: EP0001578
(87) Internationale Veröffentlichungsnummer: WO00053546

(56) Entgegenhaltungen:
- DE-A- 4 339 713

## Beschreibung

Die Erfindung betrifft ein Verfahren zur Oligomerisierung von C₆-Olefinen, insbesondere zur Herstellung von C₁₂-Olefinen durch Dimerisierung.

Verfahren zur Oligomerisierung von Olefinen sind bekannt. In der DE-A-43 39 713 ist ein Verfahren zur Oligomerisierung von Olefinen zu hochlinearen Oligomeren beschrieben. Dabei werden C₂₋₆-Olefine an einem Festbettkatalysator bei erhöhtem Druck und erhöhter Temperatur umgesetzt, wobei der eingesetzte Katalysator als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält.

In der US 4,959,491 ist ein Verfahren zur Dimerisierung von C₆-Olefinen zu C₁₂-Olefinen beschrieben, die zur Herstellung von Tensiden eingesetzt werden können. Als Katalysatoren werden nickelhaltige Katalysatoren wie Hexafluoracetoacetylnickelcyclooctadien eingesetzt.

In der DE-A- 39 14 817 ist ein Verfahren zur Oligomerisierung von C₂₋₈-Olefinen beschrieben, wobei die Umsetzung an nickelausgetauschtem Montmorillonit, einem Nickel-Aluminium-Siliciumoxid-Katalysator oder mit Nickel imprägnierten Molekularsieben oder Zeolithen durchgeführt wird. Das eingesetzte Olefingemisch wird vor der katalytischen Umsetzung über ein Molekularsieb geleitet.

Die bekannten Verfahren weisen den Nachteil einer häufig zu geringen Katalysatorstandzeit aut. Der Katalysator wird insbesondere durch höhere Oligomere zugesetzt und verliert deshalb seine Aktivität.

Aufgabe der vorliegenden Erfindung ist die Bereitstellung eines Verfahrens zur Oligomerisierung von C₆-Olefinen, das die Nachteile der bekannten Verfahren vermeidet.

Die Aufgabe wird erfindungsgemäß gelöst durch ein Verfahren zur Oligomerisierung von C₆-Olefinen durch Umsetzung eines C₆-Olefine enthaltenden Reaktionsgemisches an einem Nickel enthaltenden Festbettkatalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält, wobei die Umsetzung am Festbettkatalysator mit einem Umsatz zu oligomerisierten C₆-Olefinen, bezogen auf das Reaktionsgemisch, von maximal 30 Gew.-% gefahren wird.

Dabei wird die Umsetzung am Festbettkatalysator vorzugsweise mit einem Umsatz von 10 bis 30 Gew.-%, besonders bevorzugt 10 bis 25 Gew.-%, bezogen auf das Reaktionsgemisch, durchgeführt. Die Oligomerisierung ist dabei vorzugsweise im wesentlichen eine Dimerisierung.

Es wurde erfindungsgemäß gefunden, daß die Deaktivierung des Katalysators vermieden werden kann und die Dimer-Selektivität erhöht werden kann, wenn der Umsatz am Katalysator im angegebenen Bereich liegt. Dabei kann das Verfahren diskontinuierlich oder kontinuierlich durchgeführt werden. Vorzugsweise wird es kontinuierlich in flüssiger Phase durchgeführt. Der Umsatz bezieht sich dann auf einen Durchgang des Reaktionsgemisches durch den Katalysator.

Die Umsetzung wird vorzugsweise bei einer Temperatur im Bereich von 30 bis 300°C und einem Druck im Bereich von 10 bis 300 bar durchgeführt.

Um einen hohen Gesamtumsatz im Verfahren zu erreichen, kann ein Teil des erhaltenen umgesetzten Reaktionsgemisches nach Abtrennung der Oligomeren in die Umsetzung zurückgeführt werden. Durch Einstellung der rückgeführten Menge des Umsetzungsgemisches können sehr hohe Gesamtumsätze erreicht werden. Der Begriff "Oligomere" beinhaltet auch Dimere und höhersiedende Verbindungen.

Mit dem erfindungsgemäßen Verfahren ist es möglich, einen Gesamtumsatz von über 90% bei einer gleichzeitigen C₁₂-Selektivität von über 80% zu verwirklichen. Durch die Einhaltung des erfindungsgemäßen Umsatzes am Katalysator selbst (bezogen auf den einmaligen Durchgang) werden die Lebensdauer und Standzeit des Katalysators stark erhöht, da die Bildung höhersiedender Verbindungen unterdrückt wird, die sich auf dem Katalysator ablagern und damit einen Aktivitätsrückgang bewirken können.

Erfindungsgemäß einsetzbare C₆-Olefine können großtechnisch mittels Verfahren wie der Propylendimerisierung synthetisiert werden. Die wichtigsten industriell ausgeübten Propylendimerisierungsverfahren sind z.B. in A. Chauvel und G. Lefebvre, Petrochemical Process, Edition Technip (1989), S.183 bis 187 und F. Asinger, Die petrochemische Industrie, Akademier-Verlag (1971), S.278 bis 299 angegeben. Die Oligomerisierung wird großtechnisch entweder homogen- oder heterogenkatalytisch ausgeführt. Die heterogenen Katalysatoren, die zum Einsatz kommen können, sind z.B. in C.T. O'Connor et al., Catalysis Today Vol. 6 (1990), S.329 bis 349, aufgelistet.

Das - bezogen auf die produzierte Menge - wichtigste homogenkatalytische Verfahren ist das Dimerol-G-Verfahren von IFP. Es ist in Erdöl, Erdgas und Kohle, Heft 7/8, Juli/August 1990, S. 309 bis 315 eingehend beschrieben. Das mittels diesem Verfahren gewonnene Produkt (s.g. "Dimate") hat folgende durchschnittliche Olefin-Zusammensetzung:

| | |
|---|---|
| C₃ | 4 Gew.-% |
| C₆ | 73 Gew.-% |
| C₉ | 17 Gew.-% |
| C₁₂ | 4 Gew.-% |
| C₁₅₊ | 2 Gew.-% |

Die C₆-Fraktion setzt sich aus:

| | |
|---|---|
| 4-Methylpenten-1 | 0,9 Gew.-% |
| 2,3-Dimethylbuten-1 | 2,3 Gew.-% |
| 4-Methylpenten-2 cis | 3,1 Gew.-% |
| 4-Methylpenten-2 trans | 21,7 Gew.-% |
| 2-Methylpenten-1 | 5,0 Gew.-% |
| Hexen-1 | 0,3 Gew.-% |
| Hexen-3 trans | 4,4 Gew.-% |
| Hexen-3 cis | 0,7 Gew.-% |
| Hexen-2 trans | 13,6 Gew.-% |
| 2-Methylpenten-2 | 39,2 Gew.-% |
| Hexen-2 cis | 3,7 Gew.-% |
| 2,3-Dimethylbuten-2 | 4,8 Gew.-% |

zusammen.

Eine andere Quelle hinsichtlich C₆-Olefinen bieten Metatheseverfahren.

Als Katalysatoren kommen allgemein an sich bekannte, eine geringe Verzweigung bewirkende, Nickel enthaltende Katalysatoren in Betracht, wie sie z.B. in Catalysis Today Vol. 6 (1990), S. 336 bis 338, DE-A 43 39 713, US 5,169,824, DD 2 73 055, DE-A-20 51 402, EP-A-0 202 670, Appl. Catal. 31 (1987), Seite 259-266, EP-A- 0 261 730, NL 8 500 459, DE-A-23 47 235, US 5,134,242, EP-A-0 329 305, US 5,146,030, US 5,073,658, US 5,113,034 und US 5,169,824 beschrieben sind.

Nach dem erfindungsgemäßen Verfahren wird die Oligomerisierung in flüssiger Phase unter Verwendung der in DE-A 43 39 713 beschriebenen Katalysatoren durchgeführt.

Die dort beschriebenen Katalysatoren bestehen im wesentlichen aus Nickeloxid, Siliciumoxid, Titanoxid und/oder Zirkonoxid sowie gegebenenfalls Aluminiumoxid mit einem Gehalt an Nickeloxid von 10 bis 70 Gew.-%, 5 bis 30 Gew.-% Titandioxid und/oder Zirkondioxid, 0 bis 20 Gew.-% Aluminiumoxid und als Rest Siliciumdioxid, erhältlich durch Fällung der Katalysatormasse bei pH 5 bis 9 durch Zugabe einer Nickelnitrat enthaltenden wäßrigen Lösung zu einer Alkaliwasserglaslösung, die Titanoxid und/oder Zirkondioxid enthält, Filtrieren, Trocknen und Tempern bei 350 bis 650°C.

Die Katalysatoren bestehen vorzugsweise im wesentlichen aus 10 bis 20 Gew.-% Titandioxid, 0 bis 10 Gew.-% Aluminiumoxid, 40 bis 60 Gew.-% Nickeloxid als Hauptbestandteil und als Aktivkomponente und als Rest Siliciumdioxid.

Speziell bevorzugte Katalysatoren haben die Zusammensetzung 50 Gew.-% NiO, 34 Gew.-% SiO₂, 3 Gew.-% Al₂O₃ und 13 Gew.-%TiO₂. Sie sind weitestgehend alkalifrei (Anteil an Na₂O < 0,3 Gew.-%).

Die Katalysatoren sind bevorzugt in einem Festbett angeordnet und liegen deshalb in stückiger Form, z.B. in Form von Tabletten (5mm x 5mm, 5mm x 3mm, 3mm x 3mm), Ringen (7mm x 7mm x 3mm, 5mm x 5mm x 2mm, 5mm x 2mm x 2mm) oder Strängen (1,5mm-Durchmesser, 3mm-Durchmesser, 5mm-Durchmesser) vor.

Vorzugsweise führt man das erfindungsgemäße Verfahren so aus, daß man einen n-Hexen und/oder Methylpenten enthaltenden Kohlenwasserstoffstrom, bevorzugt in flüssiger Phase, über dem genannten Ni enthaltenden Katalysatoren umsetzt.

Geeignete C₆-Kohlenwasserstoffe sind z.B. Gemische mit folgender

| Zusammensetzung: | |
|---|---|
| Paraffin | 10 bis 90 Gew.-% |
| Olefin | 10 bis 90 Gew.-% |
| wobei die Olefin-Fraktion folgende Zusammensetzung haben kann: | |
| n-Hexene | vorzugsweise 0,1 bis 99,8 Gew.-% |
| Methylpentene | vorzugsweise 0,1 bis 99,8 Gew.-% |
| Dimethylbutene | vorzugsweise 0,1 bis 99,8 Gew.-% |

Die eingesetzten Kohlenwasserstoffströme werden zweckmäßig in an sich aus der DE-A 39 14 817 bekannter Weise mit Hilfe eines Schutzbettes wie einem Molekularsieb, Aluminiumoxiden, aluminiumoxidhaltigen Feststoffen, Aluminiumphosphaten, Siliciumdioxiden, Kieselgur, Titandioxiden, Zirkoniumdioxiden, Phosphaten, kohlenstoffhaltigen Adsorbentien, Polymeradsorbentien oder Mischungen davon von sauerstoffhaltigen Verbindungen wie Alkoholen, Aldehyden, Ketonen oder Ethern durch Adsorption befreit.

Die Oligomerisierungsreaktion findet bei Temperaturen von 30 bis 300°C, vorzugsweise von 80 bis 250°C und insbesondere von 100 bis 200°C und einem Druck von 10 bis 300 bar, vorzugsweise von 15 bis 100 bar und insbesondere von 20 bis 70 bar statt. Der Druck wird dabei zweckmäßig so ausgewählt, daß bei der eingestellten Temperatur das Einsatzgemisch flüssig vorliegt. Der Reaktor ist in der Regel ein mit dem Katalysator beschickter zylindrischer Reaktor bzw. Schachtofen, der von dem flüssigen Reaktionsgemisch von oben nach unten durchströmt wird. Nach dem Verlassen der ein- oder mehrstufigen Reaktionszone werden die gebildeten Oligomere in an sich bekannter Weise von den nicht umgesetzten C₆-Kohlenwasserstoffen getrennt (z.B. destillativ) und (letztere) vollständig oder zum größten Teil zurückgeführt (ein gewisser Purge zum Ausschleusen von Inerten, z.B. Hexan, ist jedoch immer notwendig).

Eine Besonderheit der erfindungsgemäßen Reaktionsführung besteht in der Möglichkeit, das Verfahren in einem Schachtofen adiabatisch auszuüben, da die Wärmetönung im Reaktor durch das Verdünnen der Hexene mit dem zurückgeführten Strom durch Wahl der Menge und Temperatur dieses Stromes beliebig kontrolliert werden kann. Die adiabatische Fahrweise führt im Vergleich zu einem isotherm beschriebenen Verfahren zu einem beträchtlichen Herabsetzen der Apparate-Investitionskosten.

Gemäß einer Ausführungsform der Erfindung kann das Einsatzgemisch vor der Umsetzung in einer Kolonne (K) zur Trennung von C₆-Olefinen und Oligomeren (C₇₊-Kohlenwasserstoffe) fraktioniert werden, die C₆-Olefine in die Umsetzung (Cl) geführt werden, das umgesetzte Gemisch in die Kolonne (K1) zurückgeführt werden und die Oligomere (C₇₊-Kohlenwasserstoffe) ausgeschleust werden.

Gemäß einer weiteren Ausführungsform kann das umgesetzte Gemisch nach der Umsetzung in einer Kolonne (K1) zur Trennung von C₆-Olefinen und Oligomeren fraktioniert werden, die C₆-Olefine in die Umsetzung (Cl) zurückgeführt werden, und die Oligomere ausgeschleust werden.

### Die beiden vorstehend genannten Verfahren sind in der beigefügten

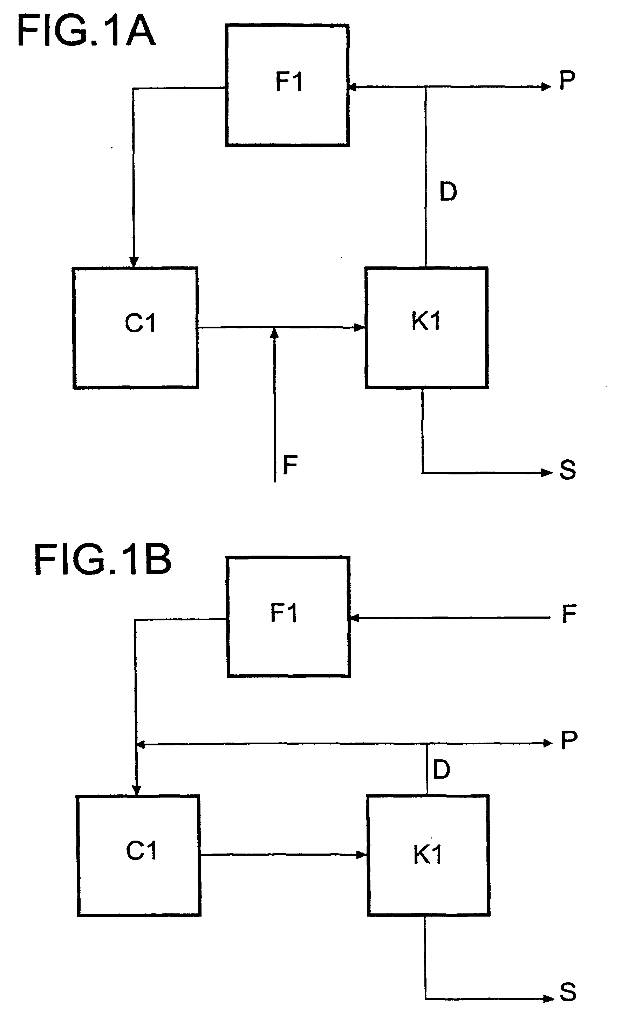

Zeichnung in Figur 1a) und b) schematisch abgebildet.

Dabei bedeuten die Bezugszeichen folgendes:
F1: Schutzbett
C1: Reaktor
K1: Kolonne
F: Feed
P: Purge
D: Destillat
S: Sumpf

Das Schutzbett (F1) dient dabei zur Entfernung von Katalysator-Giften (im wesentlichen S-N-O-haltige Kohlenwasserstoffe).

Die Auftrennung der Oligomeren erfolgt in an sich bekannter Weise durch fraktionierte Destillation zur Abtrennung der gewünschten Dodecene. Die schwefelfreie C₁₃+-Fraktion zeigt einen hohen Blend-Wert hinsichtlich Beimischung in das Dieselkraftstoff-Pool. Besonders bevorzugt ist der Einsatz dieser C₁₃+-Fraktion als Dieselkraftstoff-Mischkomponente, nachdem die Olefine durch eine Hydrierung zu Paraffinen umgesetzt wurden. Aus dieser Maßnahme ergibt sich eine Erhöhung der Cetanzahl, die für die Eigenschaften dieses Dieselkraftstoffes maßgebend ist. Zur Hydrierung kommen alle im Stand der Technik bekannten Verfahren in Frage.

Die aus der Hexendimerisierung gewonnenen Dodecene können zu Tensiden weiterverarbeitet werden.

Die folgenden Beispiele erläutern das erfindungsgemäße Verfahren näher.

### Beispiele

Die Versuchsanlage umfaßt folgende Anlagenteile (Verfahrensschema gemäß Fig.**1**):
- Adsorber zur Entfernung von Katalysatorgiften (F1, Volumen: ca. 501)
- Adiabatischer Reaktor (C1, Volumen: ca. 401, Länge: 8m, Durchmesser: 80mm)
- Destillationskolonne (Kl) zur Trennung von unumgesetzten C₆-Olefinen und den gebildeten Oligomeren [C₁₂].

Als Katalysator diente ein Material, das gemäß DE-A 43 39 713 zu 5 mm x 5 mm Tabletten hergestellt wurde. Zusammensetzung in Gew.-% der Aktivkomponenten: 50 Gew.-% NiO, 13 Gew.-% TiO₂, 34 Gew.-% SiO₂, 3 Gew.-% Al₂O₃.

Als Adsorber diente ein hochoberflächiges Aluminiumoxid wie Selexsorb® von Alcoa.

### Beispiel 1

Als Einsatzgemisch wurde ein Kohlenwasserstoffgemisch mit folgender Zusammensetzung verwendet:

| | |
|---|---|
| C₃ | 4 Gew.-% |
| C₆ | 73 Gew.-% |
| C₉ | 17 Gew.-% |
| C₁₂ | 4 Gew.-% |
| C₁₅₊ | 2 Gew.-% |

Die C₆-Fraktion setzt sich aus:

| | |
|---|---|
| 4-Methylpenten-1 | 0,9 Gew.-% |
| 2,3-Dimethylbuten-1 | 2,3 Gew.-% |
| 4-Methylpenten-2 cis | 3,1 Gew.-% |
| 4-Methylpenten-2 trans | 21,7 Gew.-% |
| 2-Methylpenten-1 | 5,0 Gew.-% |
| Hexen-1 | 0,3 Gew.-% |
| Hexen-3 trans | 4,4 Gew.-% |
| Hexen-3 cis | 0,7 Gew.-% |
| Hexen-2 trans | 13,6 Gew.-% |
| 2-Methylpenten-2 | 39,2 Gew.-% |
| Hexen-2 cis | 3,7 Gew.-% |
| 2,3-Dimethylbuten-2 | 4,8 Gew.-% |

zusammen.

Das Kohlenwasserstoffgemisch wurde mit einer Rate von 5,1 kg/h in die Kolonne K1 eingeleitet (Abb.1). Folgende Bedingungen wurden in der Versuchsanlage eingestellt:

| Adsorptionsteil: | |
|---|---|
| Druck (bar) | 15 |
| Temperatur (°C) | 35 |
| Durchsatz (kg/h) | 18,8 |

| Syntheseteil: | |
|---|---|
| Katalysatormenge (kg) | 25 |
| Druck (bar) | 15 |
| Eintrittstemperatur (°C) | 100 |
| Austrittstemperatur (°C) | 139 |
| Durchsatz (kg/h) | 18,8 |

| Destillationsteil: | |
|---|---|
| Druck (bar) | 1 |
| Temperatur - Kopf (°C) | 35 |
| Temperatur - Sumpf (°C) | 185 |
| Einsatzmenge (kg/h) | 23,9 |
| Destillat (kg/h) | 19,0 |
| Purge (kg/h) | 0,2 |
| Sumpf (kg/h) | 4,9 |

Folgendes Ergebnis wurde erzielt:

| Zusammensetzung | | | | | | |
|---|---|---|---|---|---|---|
| Strom | C₃ | C₆ | C₉ | C₁₂ | C₁₅₊ | *Summe* C₉₊ |
| Einsatzgemisch K1 =Reaktoraustrag | 1,7 | 78,1 | 3,7 | 13,4 | 3,1 | 20,2 |
| Destillat K1 | 2,1 | 97,9 | <0,1 | <0,1 | <0,1 | - |
| Sumpf K1 | <0,1 | 0,4 | 17,7 | 64,7 | 17,2 | 99,6 |

Daraus ergibt sich ein C₆-Olefin- Umsatz von 94,7% und eine C₁₂-Selektivität von 83,6% (bezogen auf die umgesetzten C₆-Olefine).

### Beispiel 2

Als Einsatzgemisch wurde ein Kohlenwasserstoffgemisch mit folgender Zusammensetzung verwendet:

| | |
|---|---|
| C₅ | 0,1 Gew.-% |
| C₆ | 98,7 Gew.-% |
| C₇ | 1,2 Gew.-% |

Die C₆-Fraktion setzt sich aus:

| | |
|---|---|
| 4-Methylpenten-1 | <0,1 Gew.-% |
| 2,3-Dimethylbuten-1 | <0,1 Gew.-% |
| 4-Methylpenten-2 cis | <0,1 Gew.-% |
| 4-Methylpenten-2 trans | <0,1 Gew.-% |
| 2-Methylpenten-1 | <0,1 Gew.-% |
| Hexen-1 | <0,1 Gew.-% |
| Hexen-3 trans | 90 Gew.-% |
| Hexen-3 cis | 10 Gew.-% |
| Hexen-2 trans | <0,1 Gew.-% |
| Hexen-2 cis | <0,1 Gew.-% |
| 2-Methylpenten-2 | <0,1 Gew.-% |
| 2,3-Dimethylbuten-2 | <0,1 Gew.-% |

zusammen.

Das Kohlenwasserstoffgemisch wurde mit einer Rate von 3,20 kg/h in den Filter F1 eingeleitet (Abb.2). Folgende Bedingungen wurden in der Versuchsanlage eingestellt:

| Adsorptionsteil: | |
|---|---|
| Druck (bar) | 10 |
| Temperatur (°C) | 35 |
| Durchsatz (kg/h) | 3,20 |

| Syntheseteil: | |
|---|---|
| Katalysatormenge (kg) | 25 |
| Druck (bar) | 10 |
| Eintrittstemperatur (°C) | 100 |
| Austrittstemperatur (°C) | 133 |
| Durchsatz (kg/h) | 15,75 |

| Destillationsteil: | |
|---|---|
| Druck (bar) | 1 |
| Temperatur - Kopf (°C) | 45 |
| Temperatur - Sumpf (°C) | 182 |
| Einsatzmenge (kg/h) | 15,75 |
| Destillat (kg/h) | 12,60 |
| Purge (kg/h) | 0,05 |
| Sumpf (kg/h) | 3,15 |

Folgendes Ergebnis wurde erzielt:

| Zusammensetzung | | | | | | |
|---|---|---|---|---|---|---|
| Strom | C₅ | C₆ | C₇₋₁₁ | C₁₂ | C₁₃₊ | *Summe* C₇₊ |
| Einsatzgemisch K1 =Reaktoraustrag | <0,1 | 80,6 | 0,4 | 15,7 | 3,3 | 19,4 |
| Destillat K1 | 0,1 | 99,9 | <0,1 | <0,1 | <0,1 | - |
| Sumpf K1 | <0,1 | 0,4 | 1,3 | 81,2 | 17,1 | 99,6 |

Daraus ergibt sich ein C₆-Olefin- Umsatz von 98,4% und eine C₁₂-Selektivität von 82,6% (bezogen auf die umgesetzten C₆-Olefine).

## Patentansprüche

1. Verfahren zur Oligomerisierung von C₆-Olefinen durch Umsetzung eines C₆-Olefine enthaltenden Reaktionsgemisches an einem Nickel enthaltenden Festbettkatalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält, **dadurch gekennzeichnet, daß** die Umsetzung am Festbettkatalysator mit einem Umsatz zu oligomerisierten C₆-Olefinen, bezogen auf das Reaktionsgemisch, von maximal 30 Gew.-% gefahren wird.

2. Verfahren nach Anspruch 1, **dadurch gekennzeichnet, daß** die Umsetzung am Festbettkatalysator mit einem Umsatz zu oligomerisierten C₆-Olefinen, bezogen auf das Reaktionsgemisch, von 10 bis 30 Gew.-% gefahren wird.

3. Verfahren nach Anspruch 1 oder 2, **dadurch gekennzeichnet, daß** die Oligomerisierung im wesentlichen eine Dimerisierung ist.

4. Verfahren nach einem der Ansprüche 1 bis 3, **dadurch gekennzeichnet, daß** es bei einem Druck im Bereich von 10 bis 300 bar und einer Temperatur im Bereich von 30 bis 300 °C durchgeführt wird.

5. Verfahren nach einem der Ansprüche 1 bis 4, **dadurch gekennzeichnet, daß** es kontinuierlich in flüssiger Phase durchgeführt wird.

6. Verfahren nach Anspruch 5, **dadurch gekennzeichnet, daß** es in einem Schachtofen adiabatisch durchgeführt wird und ein Teil des umgesetzten Gemisches in die Umsetzung zurückgeführt wird.

7. Verfahren nach einem der Ansprüche 1 bis 6, **dadurch gekennzeichnet, daß** das Einsatzgemisch vor der Umsetzung in einer Kolonne zur Trennung von C₆-Olefinen und Oligomeren fraktioniert wird, die C₆-Olefine in die Umsetzung geführt werden, das umgesetzte Gemisch in die Kolonne zurückgeführt wird und die Oligomere (C₇₊-Kohlenwasserstoffe) ausgeschleust werden.

8. Verfahren nach einem der Ansprüche 1 bis 6, **dadurch gekennzeichnet, daß** das umgesetzte Gemisch nach der Umsetzung in einer Kolonne zur Trennung von C₆-Olefinen und Oligomeren fraktioniert wird, die C₆-Olefine in die Umsetzung zurückgeführt werden und die Oligomere ausgeschleust werden.

9. Verfahren nach einem der Ansprüche 1 bis 8, **dadurch gekennzeichnet, daß** das Reaktionsgemisch vor der Umsetzung über ein Schutzbett geführt wird.

## Claims

1. A process for oligomerizing C₆-olefins by reaction of a C₆-olefin-containing reaction mixture over a nickel-containing fixed-bed catalyst comprising from 10 to 70% by weight of nickel oxide, from 5 to 30% by weight of titanium dioxide and/or zirconium dioxide and from 0 to 20% by weight of aluminum oxide as significant active constituents and silicon dioxide as the remainder, wherein the reaction over the fixed-bed catalyst is run at a conversion to oligomerized C₆-olefins of not more than 30% by weight, based on the reaction mixture.

2. A process as claimed in claim 1, wherein the reaction over the fixed-bed catalyst is run at a conversion to oligomerized C₆-olefins of from 10 to 30% by weight, based on the reaction mixture.

3. A process as claimed in claim 1 or 2, wherein the oligomerization is essentially a dimerization.

4. A process as claimed in any of claims 1 to 3 carried out at from 30 to 300°C and a pressure in the range from 10 to 300 bar.

5. A process as claimed in any of claims 1 to 4 carried out continuously in the liquid phase.

6. A process as claimed in claim 5 which is carried out adiabatically in a shaft oven and in which part of the reacted mixture is returned to the reaction.

7. A process as claimed in any of claims 1 to 6, wherein the feed mixture is fractionated in a column to separate C₆-olefins and oligomers prior to the reaction, the C₆-olefins are returned to the reaction, the reacted mixture is returned to the column and the oligomers (C₇₊-hydrocarbons) are discharged.

8. A process as claimed in any of claims 1 to 6, wherein the reacted mixture is fractionated in a column to separate C₆-olefins and oligomers after the reaction, the C₆-olefins are returned to the reaction and the oligomers are discharged.

9. A process as claimed in any of claims 1 to 8, wherein the reaction mixture is passed over a protective bed prior to the reaction.

## Revendications

1. Procédé d'oligomérisation d'oléfines en C₆ au moyen de la réaction d'un mélange contenant des oléfines en C₆ sur un catalyseur en lit fixe contenant du nickel, qui contient, en tant que principaux composants actifs, 10% à 70% en poids d'oxyde de nickel, 5% à 30% en poids de dioxyde de titane et/ou de dioxyde de zirconium, 0% à 20% en poids d'oxyde d'aluminium et le complément de dioxyde de silicium, **caractérisé en ce que** l'on conduit la réaction sur un catalyseur en lit fixe avec une conversion en oléfines en C₆ oligomérisées d'au maximum 30% en poids par rapport au mélange réactionnel.

2. Procédé selon la revendication 1, **caractérisé en ce que** l'on conduit la réaction sur un catalyseur en lit fixe avec une conversion en oléfines en C₆ oligomérisées allant de 10% à 30% en poids par rapport au mélange réactionnel.

3. Procédé selon la revendication 1 ou 2, **caractérisé en ce que** l'oligomérisation est principalement une dimérisation.

4. Procédé selon l'une quelconque des revendications 1 à 3, **caractérisé en ce que** l'on travaille sous une pression allant de 10 bars à 300 bars et à une température allant de 30°C à 300°C.

5. Procédé selon l'une quelconque des revendications 1 à 4, **caractérisé en ce que** l'on travaille en continu en phase liquide.

6. Procédé selon la revendication 5, **caractérisé en ce que** l'on travaille dans un four à cuve de manière adiabatique et que l'on recycle une partie du mélange ayant réagi dans la réaction.

7. Procédé selon l'une quelconque des revendications 1 à 6, **caractérisé en ce que** l'on fractionne le mélange réactionnel, avant la réaction, dans une colonne afin de séparer les oléfines en C₆ et les oligomères, **en ce que** l'on conduit les oléfines en C₆ dans la réaction, **en ce que** l'on recycle le mélange ayant réagi dans la colonne et **en ce que** l'on soutire les oligomères (hydrocarbures en C₇ et plus).

8. Procédé selon l'une quelconque des revendications 1 à 6, **caractérisé en ce que** l'on fractionne le mélange ayant réagi, après la réaction, dans une colonne afin de séparer les oléfines en C₆ et les oligomères, **en ce que** l'on recycle les oléfines en C₆ dans la réaction et **en ce que** l'on soutire les oligomères.

9. Procédé selon l'une quelconque des revendications 1 à 8, **caractérisé en ce que** l'on fait passer le mélange réactionnel, avant la réaction, sur un lit de protection.
